# EUROPÄISCHE PATENTSCHRIFT

(11) **EP 0 906 570 B1**
(45) Veröffentlichungstag und Bekanntmachung des Hinweises auf die Patenterteilung: **10.01.2001**
(21) Anmeldenummer: 97925972.8
(22) Anmeldetag: 30.05.1997
(51) Int. Cl.: G01N 33/52, G01N 33/70, G01N 33/72, C12Q 1/00, C12Q 1/32

(54) **VERFAHREN ZUR BESEITIGUNG VON HÄMOGLOBINSTÖRUNGEN BEI DER ANALYSE MEDIZINISCHER PROBEN**
PROCESS TO ELIMINATE HAEMOGLOBIN ERRORS WHEN ANALYSING MEDICAL SAMPLES
PROCEDE POUR SUPPRIMER LES ERREURS DUES A L'HEMOGLOBINE LORS DE L'ANALYSE D'ECHANTILLONS MEDICAUX

(30) Priorität: 31.05.1996 DE 19622090
(43) Veröffentlichungstag der Anmeldung: 07.04.1999
(73) Patentinhaber: Roche Diagnostics GmbH, 68305 Mannheim (DE)
(72) Erfinder: WEISHEIT, Ralph, 82380 Peissenberg (DE); PFITSCHLER, Elke, D-82386 Oberhausen (DE)
(74) Vertreter: Weiss, Wolfgang, Dipl.-Chem. Dr.
(86) Internationale Anmeldenummer: EP9702835
(87) Internationale Veröffentlichungsnummer: WO9745733

(56) Entgegenhaltungen:
- EP-A- 0 158 506
- CLINICAL CHEMISTRY, Bd. 25, Nr. 6, 1979, WINSTON-SALEM SC USA, Seiten 951-959, XP002037050 B. HAHN ET AL.: "Polychromatic analysis: new applications of an old technique." in der Anmeldung erwähnt
- EUROPEAN JOURNAL OF CLINICAL CHEMISTRY AND CLINICAL BIOCHEMISTRY, Bd. 31, Nr. 9, 1993, BERLIN FRG, Seiten 595-601, XP002037051 F. DA FOSECA-WOLLHEIM : "Hemoglobin interference in the bichromatic spectrophotometry of NAD(P)H at 340/380 nm. " in der Anmeldung erwähnt
- ANONYMOUS: "DIAGNOSTIK KATALOG", 1997, BOEHRINGER MANNHEIM DIAGNOSTIKA, MANNHEIM BRD
- ANONYMOUS:"Geräteinstellungen für das Boehringer Mannheim/Hitachi 717-Analysegerät", 01. Juni 1996, BOEHRINGER MANNHEIM, MANNHEIM BRD

## Beschreibung

Die Erfindung betrifft ein Verfahren zur Bestimmung eines Analyten in einer freies Hämoglobin enthaltenden Probe, wobei die Bestimmung durch eine optische bichromatische Messung bei einer Haupt- und einer Nebenmeßwellenlänge erfolgt. Insbesondere ist dieses Verfahren zur Bestimmung der Parameter Ammoniak, Creatinkinase und Isoenzymen davon und Lactat-Dehydrogenase und Isoenzymen davon in einer medizinischen Probe, z. B. einer Serum- oder Plasmaprobe, geeignet.

Es ist allgemein bekannt, daß durch Hämolyse die Bestimmung einer Vielzahl von Analyten in teilweise erheblichem Maße gestört ist. Um dennoch unverfälschte Meßwerte zu erhalten, wurden in der Vergangenheit unterschiedliche Verfahren zur Hämolyse-Entstörung publiziert.

Eines dieser Verfahren besteht darin, daß bei der Messung in automatisierten Analysegeräten neben der ersten Meßwellenlänge (Hauptmeßwellenlänge) häufig eine zweite Meßwellenlänge (Nebenmeßwellenlänge) benutzt wird, mittels derer der Störeinfluß interferierender Substanzen wie Hämoglobin, Bilirubin und Lipämie beseitigt oder zumindest minimiert werden kann.

Eine Anforderung dabei ist, daß die zu messende Substanz bei der Nebenwellenlänge möglichst wenig absorbiert, die Störsubstanz dagegen möglichst in gleicher Höhe absorbiert wie bei der Hauptwellenlänge (Praxistechnik: Photometer für die Ärztliche Praxis, Deutscher Ärzteverlag 1977, Seiten 41-42).

Im DIA-Letter (Boehringer Mannheim) Nr. 70 (1985) wird erwähnt, daß die Nebenwellenlänge möglichst nahe der Hauptwellenlänge liegen sollte, da in der Regel dann die Störsubstanz bei Haupt- und Nebenwellenlänge ähnliche Extinktionen aufweist.

In Clin Chem 25/6, 951-959 (1979) wird darauf verwiesen, die Nebenwellenlänge so zu wählen, daß diese nahe des Absorptionsminimums des Chromogens und nahe des Absorptionsmaximums der Störsubstanz liegen sollte. In diesem Zusammenhang wird für die Glucosebestimmung (Hauptwellenlänge 340 nm) eine Nebenwellenlänge von 380 nm empfohlen, da hier die Störsubstanzen ähnlich absorbieren wie bei 340 nm.

Kritisch wird dagegen in Eur J Clin Chem Clin Biochem 31/9, 595-601 (1993) gesehen, UV-Tests mit einer Nebenwellenlänge von 380 nm zu vermessen, da in diesem Fall die Umwandlung von Hb-O₂ in Meth-Hb zu spektralen Veränderungen bei 380 nm und damit zu Fehlmessungen führt. Für Tests, die auf Messung von NAD(P)H-Abnahme oder -Zunahme beruhen, wird deshalb eine Nebenwellenlänge empfohlen, die jenseits der sogenannten Soret-Region liegt, wie z. B. 475 nm.

Alle zuvor beschriebenen Verfahren beziehen sich auf die Entstörung von durch Hämolyse verursachten Fehlmessungen. Mit der Bereitstellung von Blutersatzmitteln auf Basis von Hämoglobin stellt sich die Frage nach der Beseitigung von Störungen durch natives oder synthetisches Hämoglobin bzw. Hb-analogen Verbindungen noch weit brisanter als bisher. Solche Störungen treten dann nämlich einerseits auch in nicht hämolytischem Probenmaterial und andererseits auch in weit höherem Grad auf als bei nativer Hämolyse, da bei Therapie mit Blutersatzmitteln der Hb-Gehalt im Blutserum oder -plasma bis zu 2.000 mg/dl betragen kann.

Außerdem wurde festgestellt, daß bei der Messung bestimmter Analyten, wie etwa Ammoniak, Creatinkinase und Isoenzymen davon sowie Lactat-Dehydrogenase und Isoenzymen davon die Verwendung einer Nebenmeßwellenlänge von 475 nm oder höher, z. B. bei 480, 505, 600, 660 oder 700 nm, nicht ohne weiteres eine ausreichende Hämoglobinentstörung erzielt. Da diese Parameter im Rahmen der Herz-Kreislauf- und Notfalldiagnostik sowie der Diagnostik von mit Blutersatzmitteln therapierten Patienten von essentieller Bedeutung sind, war es die Aufgabe der vorliegenden Erfindung, ein einfaches Verfahren zur Beseitigung von Störungen bereitzustellen, die durch natives Hämoglobin oder auf synthetischem Hb- bzw. Hb-analogen Verbindungen basierenden Blutersatzmitteln, insbesondere bei Messung der oben genannten Analyten hervorgerufen werden.

Gelöst wurde die der Erfindung zugrunde liegende Aufgabe durch ein Verfahren zur Bestimmung eines Analyten in einer freies Hämoglobin enthaltenden Probe durch optische bichromatische Messung bei einer Haupt- und einer Nebenmeßwellenlänge, wobei daß man eine Nebenmeßwellenlänge von oberhalb 475 nm verwendet, bei der sich Absorptionsbanden von Hämoglobin befinden.

Bevorzugte Nebenmeßwellenlängen für das erfindungsgemäße Verfahren liegen im Bereich von 546 ± 10 nm, insbesondere 546 ± 5 nm sowie im Bereich von 570 ± 10 nm und insbesondere 570 ± 5 nm. Am meisten bevorzugt sind die Wellenlängen 546 bzw. 570 nm.

Die Auswahl der erfindungsgemäßen Wellenlängen als Nebenmeßwellenlängen war überraschend, da bei der aus dem Stand der Technik (beispielsweise Geräteeinstellungen für das Boehringer Mannheim/Hitachi 717-Analysegerät laut Empfehlungen der Pakkungsbeilage für Reagenz zur Bestimmung von Creatinkinase, Bestellnr. 1 273 248, Boehringer Mannheim Diagnostica Katalog 1997) bekannten Nebenmeßwellenlänge von 405 nm, bei welcher ebenfalls eine Absorption durch Hämoglobin stattfindet, gerade die größten Störungen durch Hämoglobin erhalten werden. Weiterhin wird in der oben genannten Publikation Eur J Clin Chem Clin Biochem darauf hingewiesen, daß zur Entstörung von Hämoglobin gerade eine Nebenmeßwellenlänge verwendet werden sollte, die jenseits der Soret-Region (der Hauptabsorptionsbande von Hämoglobin) liegt, so daß es allenfalls naheliegend wäre, als Nebenmeßwellenlänge solche Wellenlängen zu wählen, wo sich überhaupt keine Absorptionsbanden von Hämoglobin befinden.

Die erfindungsgemäße Entstörungsmethode ist für Verfahren geeignet, bei denen die Bestimmung des Analyten durch optische Messung erfolgt, insbesondere durch optische Messung bei einer Hauptwellenlänge im UV-Bereich. Besonders bevorzugt wird das Verfahren bei Tests durchgeführt, die auf einer Messung der Zu- oder Abnahme der Konzentration von NADH oder NADPH in der Probe beruhen. In diesem Fall verwendet man bevorzugt eine Hauptmeßwellenlänge im Bereich von 340 ± 10 nm.

Das erfindungsgemäße Verfahren eignet sich zur Bestimmung beliebiger Proben, in denen freies Hämoglobin vorliegt. Beispiele für solche Proben sind hämolytische Serum- oder Plasmaproben oder Proben, die ein Blutersatzmittel enthalten. Beispiele für Blutersatzmittel, die im Sinne der vorliegenden Erfindung unter den Begriff "freies Hämoglobin" fallen, sind derivatisierte, polymerisierte, modifizierte oder quervernetzte Derivate von Hämoglobinen, insbesondere von Humanhämoglobin oder Rinderhämoglobin, z. B. DCL-Hämoglobin (Diaspirincrosslinked-Hämoglobin), sowie rekombinant hergestelltes Hämoglobin.

In einer bevorzugten Ausführungsform des erfindungsgemäßen Verfahrens bestimmt man den Gehalt eines Analyten, ausgewählt aus der Gruppe bestehend aus Ammoniak, Creatinkinase und Isoenzymen davon und Lactat-Dehydrogenase und Isoenzymen davon.

Die Bestimmung von Ammoniak durch das erfindungsgemäße Verfahren erfolgt vorzugsweise nach der enzymatischen UV-Methode (Da Fonseca-Wollheim F., Z.Klin.Chem.Klin.Biochem. 11 (1973) 421).

Die Bestimmung der Creatinkinase (CK) erfolgt vorzugsweise nach der "Optimierten Standard-Methode" der Deutschen Gesellschaft für klinische Chemie (J.Clin.Chem.Clin.Biochem. 15 (1977), 249). Die Bestimmung des Creatinkinase-Isoenzyms CK-MB erfolgt vorzugsweise nach der immunologischen UV-Methode (Würzburg U. et al., Klin. Wschr. 54 (1976), 357).

Die Bestimmung von Lactat-Dehydrogenase (LDH) oder des Lactat-Dehydrogenase Isoenzyms (HBDH) (1-Hydroxybutyrat-Dehydrogenase) erfolgt vorzugsweise nach der "Optimierten Standard-Methode" der Deutschen Gesellschaft für Klinische Chemie (Z.Klin. Chem.Klin.Biochem. 8 (1970), 658 und 10 (1972), 182).

Als Probe wird beim erfindungsgemäßen Verfahren vorzugsweise eine Serum- oder Plasmaprobe eingesetzt, insbesondere eine humane Serum- oder Plasmaprobe.

Ein besonderer Vorteil des erfindungsgemäßen Verfahrens besteht darin, daß es in einem Analyseautomaten durchgeführt werden kann, z. B. an einem Boehringer Mannheim/Hitachi 704- oder 717-Analysegerät. Bei derartigen Analysegeräten können ohne weiteres die besonders bevorzugten Nebenmeßwellenlängen von 546 bzw. 570 nm eingestellt werden.

Weiterhin wird die Erfindung durch die nachfolgenden Beispiele erläutert:

### Allgemeine Methoden

Ein Teil eines Serumpools wurde mit einer Hämoglobin-haltigen Lösung derart versetzt, daß ein Hämoglobingehalt von 2.000 mg/dl erreicht wurde. Ein anderer, gleich großer Teil des Serumpools wurde mit der äquivalenten Menge einer NaCl-Lösung (154 mmol/l) versetzt. Beide Teile wurden anschließend in unterschiedlichem Verhältnis derart miteinander vermischt, daß eine Hb-Konzentrationsreihe aus 11 Proben entstand, wobei eine Probe kein Hb und die höchste Probe 2.000 mg/dl Hb enthielt.

### Beispiel 1

### Bestimmung von Ammoniak

Die Bestimmung wurde an einem Boehringer Mannheim/Hitachi 717-Analysegerät durchgeführt. Es wurden folgende Reagenzien verwendet:
- Reagenz 1:: 150 mmol/l Triethanolamin-Puffer, pH 8,6; 15 mmol/l α-Ketoglutarat; 1,5 mmol/l ADP
- Reagenz 2:: 150 mmol/l Triethanolamin-Puffer; pH 8,6; 15 mmol/l α-Ketoglutarat; 1,5 mmol/l ADP; 0,31 mmol/l NADPH; ≥ 24 U/ml Glutamat-Dehydrogenase (GLDH)

Die Testdurchführung war wie folgt: Zu 20 µl Probe wurden 200 µl Reagenz 1 und nach 5 min 50 µl Reagenz 2 gegeben. Die Bestimmung des Analyten erfolgte nach einer Dauer von weiteren 40 sec. Zur Messung wurden eine Hauptmeßwellenlänge von 340 nm und Nebenmeßwellenlängen von 405 nm, 480 mn, 505 nm, 600 nm, 660 nm und 700 nm (Vergleich) sowie von 546 nm und 570 nm (Erfindung) verwendet.

Das Ergebnis dieser Bestimmung ist in Tabelle 1 gezeigt. Es ist zu erkennen, daß bei Verwendung der erfindungsgemäßen Meßwellenlängen von 546 bzw. 570 nm eine deutlich verbesserte Wiederfindung (recovery) als bei den anderen Meßwellenlängen erreicht wurde.

### Beispiel 2

### Bestimmung von Creatinkinase

Die Bestimmung wurde an einem Boehringer Mannheim/Hitachi 717-Analysegerät durchgeführt. Es wurden folgende Reagenzien verwendet:
- Reagenz 1:: 110 mmol/l Imidazol-Puffer; pH 6,7; 20,5 mmol/l Glucose; 2,05 mmol/l EDTA; 2,5 mmol/l ADP; 6,1 mmol/l AMP; 12 µmol/l Diadenosinpentaphosphat; 2,5 mmol/l NADP; 25 mmol/l N-Acetylcystein; ≥ 3,1 U/ml Hexokinase (HK); ≥ 1,8 U/ml Glucose-6-phosphat-Dehydrogenase (G6P-DH)
- Reagenz 2:: 25 mmol/l Imidazol-Puffer; pH 7,5; 20,5 mmol/l Glucose; 2,05 mmol/l EDTA; 61 mmol/l Mg²⁺; 184 mmol/l Creatinphosphat

Die Testdurchführung war wie folgt: Zu 7 µl Probe wurden 250 µl Reagenz 1 und nach 5 min 50 µl Reagenz 2 gegeben. Die Bestimmung des Analyten erfolgte nach einer Dauer von weiteren 2 min. Zur Messung wurden eine Hauptmeßwellenlänge von 340 nm und Nebenmeßwellenlängen von 405 nm, 480 mn, 505 nm, 600 nm, 660 nm und 700 nm (Vergleich) sowie von 546 nm und 570 nm (Erfindung) verwendet.

Das Ergebnis dieser Bestimmung ist in Tabelle 2 gezeigt. Es ist zu erkennen, daß bei Verwendung der erfindungsgemäßen Meßwellenlängen von 546 bzw. 570 nm eine deutlich verbesserte Wiederfindung (recovery) als bei den anderen Meßwellenlängen erreicht wurde.

### Beispiel 3

### Bestimmung des Creatinkinase-Isoenzyms CK-MB

Die Bestimmung wurde an einem Boehringer Mannheim/Hitachi 717-Analysegerät durchgeführt. Es wurden folgende Reagenzien verwendet:
- Reagenz 1:: 110 mmol/l Imidazol-Puffer; pH 6,7; 21 mmol/l Glucose; 11 mmol/l Mg²⁺; 2,1 mmol/l EDTA; 2,4 mmol/l ADP; 6,0 mmol/l AMP; 12 µmol/l Diadenosinpentaphosphat; 2,4 mmol/l NADP; 24 mmol/l N-Acetylcystein; ≥ 3,0 U/ml HK; ≥ 1,8 U/ml G6P-DH; Antikörper, Hemmkapazität gegen CK-M bis 2000 U/l
- Reagenz 2:: 110 mmol/l Imidazol-Puffer; pH 6,7; 21 mmol/l Glucose; 2,1 mmol/l EDTA; 11 mmol/l Mg²⁺; 186 mmol/l Creatinphosphat

Die Testdurchführung war wie folgt: Zu 12 µl Probe wurden 250 µl Reagenz 1 und nach 5 min 50 µl Reagenz 2 gegeben. Die Bestimmung des Analyten erfolgte nach einer Dauer von weiteren 3 min. Zur Messung wurden eine Hauptmeßwellenlänge von 340 nm und Nebenmeßwellenlängen von 405 nm, 480 mn, 505 nm, 600 nm, 660 nm und 700 nm (Vergleich) sowie von 546 nm und 570 nm (Erfindung) verwendet.

Das Ergebnis dieser Bestimmung ist in Tabelle 3 gezeigt. Es ist zu erkennen, daß bei Verwendung der erfindungsgemäßen Meßwellenlängen von 546 bzw. 570 nm eine deutlich verbesserte Wiederfindung (recovery) als bei den anderen Meßwellenlängen erreicht wurde.

### Beispiel 4

### Bestimmung von Lactat-Dehydrogenase

Die Bestimmung wurde an einem Boehringer Mannheim/Hitachi 717-Analysegerät durchgeführt. Es wurden folgende Reagenzien verwendet:
- Reagenz 1:: 68 mmol/l Phosphat-Puffer; pH 7,5; ≥ 0,73 mmol/l Pyruvat
- Reagenz 2:: ≥ 1,1 mmol/l NADH

Die Testdurchführung war wie folgt: Zu 5 µl Probe wurden 250 µl Reagenz 1 und nach 5 min 50 µl Reagenz 2 gegeben. Die Bestimmung des Analyten erfolgte nach einer Dauer von weiteren 60 sec. Zur Messung wurden eine Hauptmeßwellenlänge von 340 nm und Nebenmeßwellenlängen von 405 nm, 480 mn, 505 nm, 600 nm, 660 nm und 700 nm (Vergleich) sowie von 546 nm und 570 nm (Erfindung) verwendet.

Das Ergebnis dieser Bestimmung ist in Tabelle 4 gezeigt. Es ist zu erkennen, daß bei Verwendung der erfindungsgemäßen Meßwellenlängen von 546 bzw. 570 nm eine deutlich verbesserte Wiederfindung (recovery) als bei den anderen Meßwellenlängen erreicht wurde.

### Beispiel 5

### Bestimmung des LDH-Isoenzyms HBDH

Die Bestimmung wurde an einem Boehringer Mannheim/Hitachi 717-Analysegerät durchgeführt. Es wurden folgende Reagenzien verwendet:
- Reagenz 1:: 68 mmol/l Phosphat-Puffer; pH 7,5; 3,7 mmol/l α-Oxobutyrat
- Reagenz 2:: ≥ 1,1 mmol/l NADH

Die Testdurchführung war wie folgt: Zu 5 µl Probe wurden 250 µl Reagenz 1 und nach 5 min 50 µl Reagenz 2 gegeben. Die Bestimmung des Analyten erfolgte nach einer Dauer von weiteren 60 sec. Zur Messung wurden eine Hauptmeßwellenlänge von 340 nm und Nebenmeßwellenlängen von 405 nm, 480 mn, 505 nm, 600 nm, 660 nm und 700 nm (Vergleich) sowie von 546 nm und 570 nm (Erfindung) verwendet.

Das Ergebnis dieser Bestimmung ist in Tabelle 5 gezeigt. Es ist zu erkennen, daß bei Verwendung der erfindungsgemäßen Meßwellenlängen von 546 bzw. 570 nm eine deutlich verbesserte Wiederfindung (recovery) als bei den anderen Meßwellenlängen erreicht wurde.

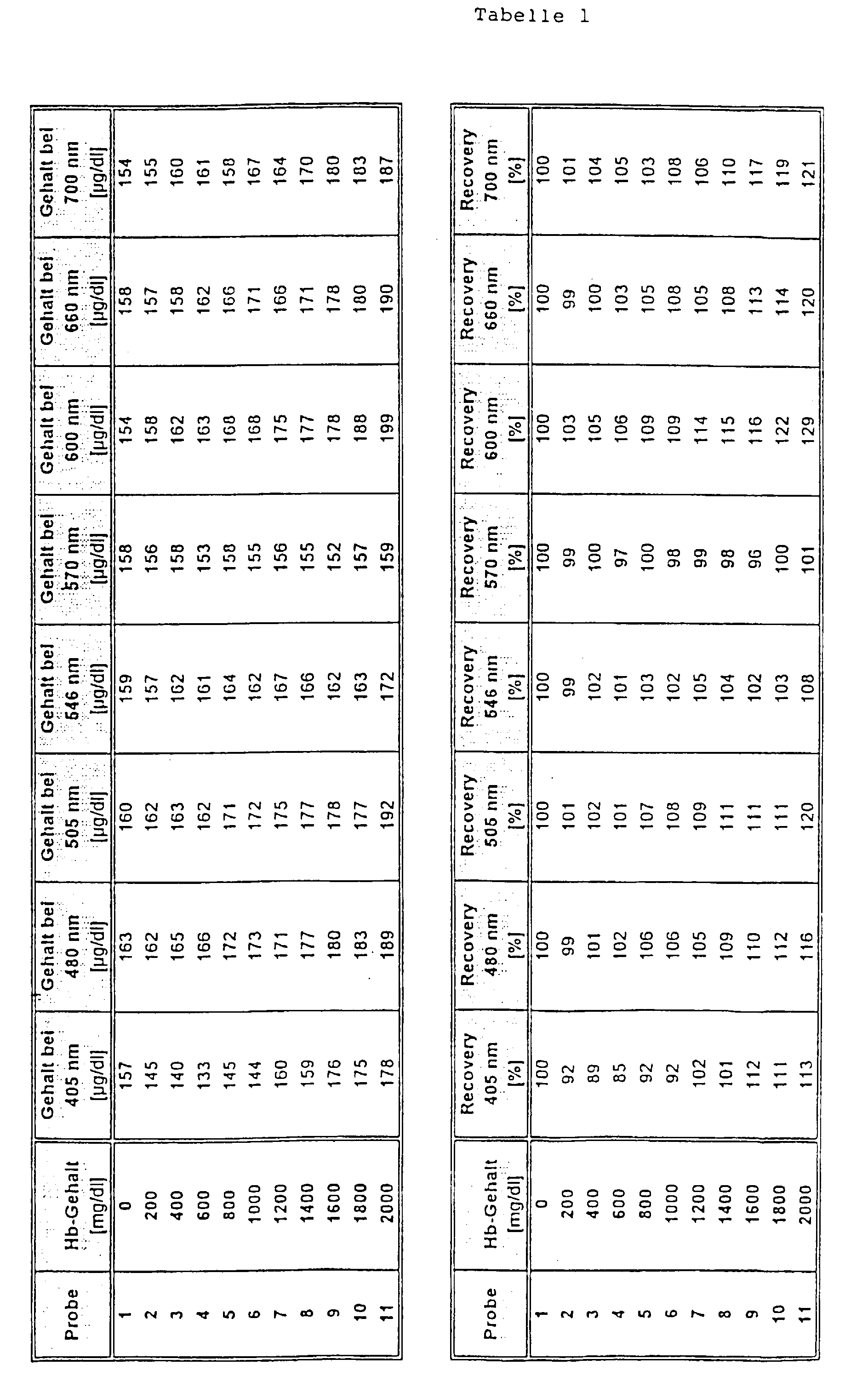

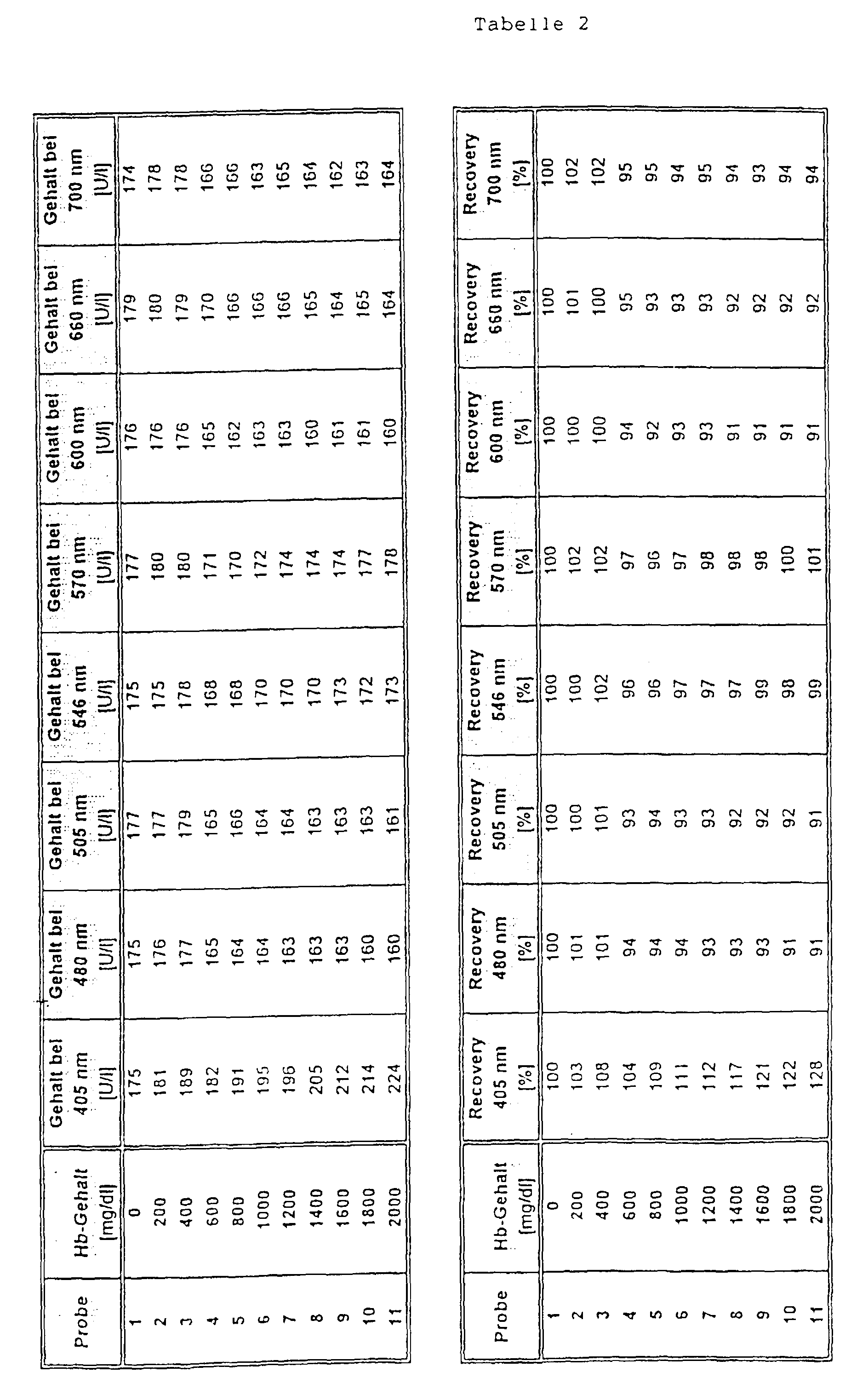

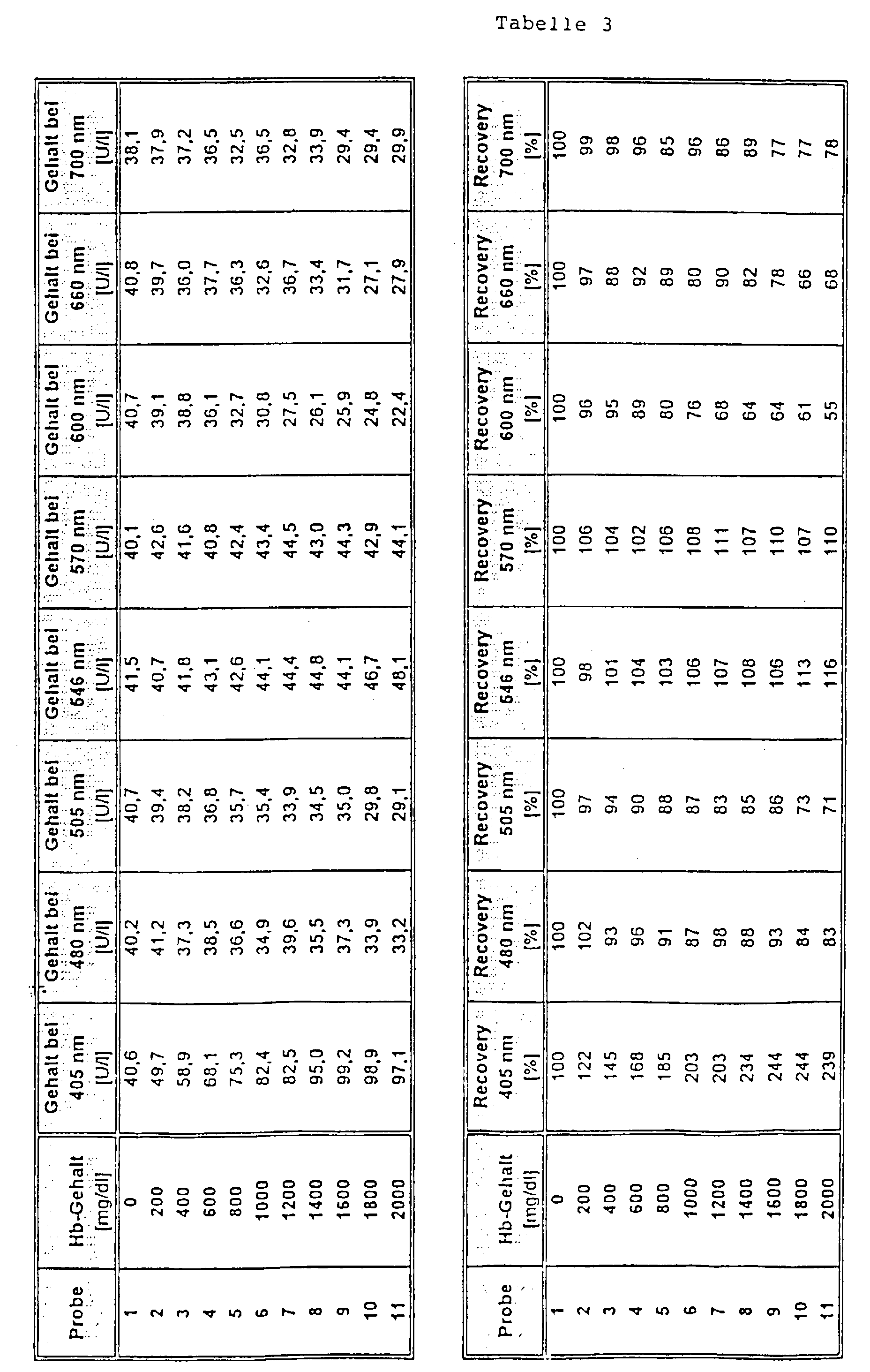

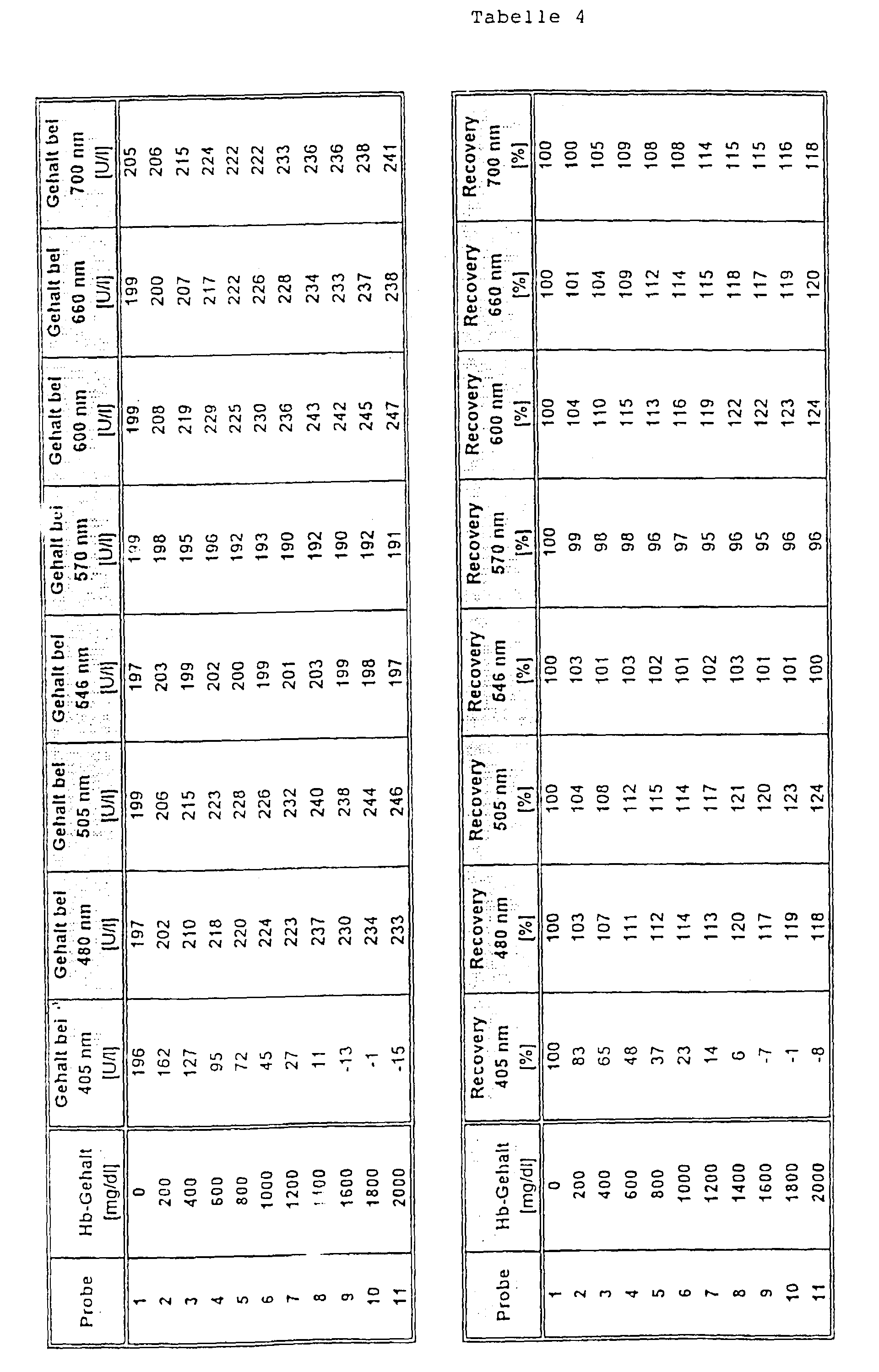

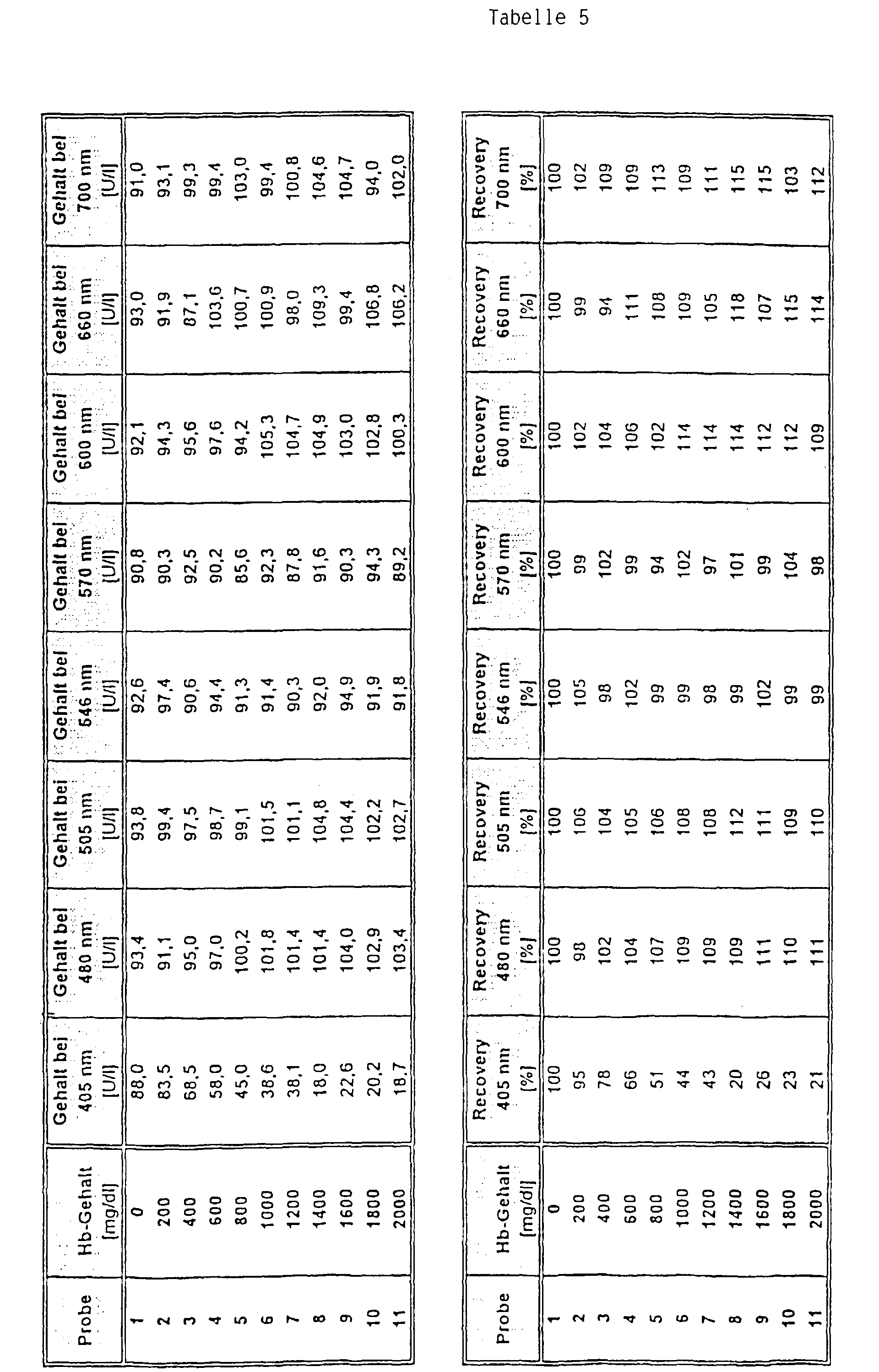

## Patentansprüche

1. Verfahren zur Bestimmung eines Analyten in einer freies Hämoglobin enthaltenden Probe durch optische bichromatische Messung bei einer Haupt- und einer Nebenmeßwellenlänge,
**dadurch gekennzeichnet,**
daß man eine Nebenmeßwellenlänge von oberhalb 475 nm verwendet, bei der sich Absorptionsbanden von Hämoglobin befinden.

2. Verfahren nach Anspruch 1,
**dadurch gekennzeichnet,**
daß man eine Nebenmeßwellenlänge im Bereich von 546 ± 10 nm verwendet.

3. Verfahren nach Anspruch 1,
**dadurch gekennzeichnet,**
daß man eine Nebenmeßwellenlänge im Bereich von 570 ± 10 nm verwendet.

4. Verfahren nach einem der Ansprüche 1-3,
**dadurch gekennzeichnet,**
daß man einen Test durchführt, der auf einer Messung der Zu- oder Abnahme der Konzentration von NADH oder NADPH in der Probe beruht.

5. Verfahren nach Anspruch 4,
**dadurch gekennzeichnet,**
daß man eine Hauptmeßwellenlänge im Bereich von 340 ± 10 nm verwendet.

6. Verfahren nach einem der Ansprüche 1-5,
**dadurch gekennzeichnet,**
daß man den Gehabt eines Analyten bestimmt, ausgewählt aus der Gruppe bestehend aus Ammoniak, Creatinkinase und Isoenzymen davon und Lactat-Dehydrogenase und Isoenzymen davon.

7. Verfahren nach Anspruch 6,
**dadurch gekennzeichnet,**
daß man eine Bestimmung von Ammoniak durchführt.

8. Verfahren nach Anspruch 6,
**dadurch gekennzeichnet,**
daß man eine Bestimmung von Creatinkinase oder/und des Creatinkinase-Isoenzyms CK-MB durchführt.

9. Verfahren nach Anspruch 6,
**dadurch gekennzeichnet,**
daß man eine Bestimmung von Lactatdehydrogenase oder/und des Lactatdehydrogenase-Isoenzyms HBDH durchführt.

10. Verfahren nach einem der Ansprüche 1-9,
**dadurch gekennzeichnet,**
daß man eine Probe bestimmt, die ein Blutersatzmittel enthält.

11. Verfahren nach einem der Ansprüche 1-10,
**dadurch gekennzeichnet,**
daß die Bestimmung an einer Serum- oder Plasmaprobe durchgeführt wird.

12. Verfahren nach einem der Ansprüche 1-11,
**dadurch gekennzeichnet,**
daß man die Bestimmung in einem Analyseautomaten durchführt.

## Claims

1. Method for the determination of an analyte in a sample containing free haemoglobin by optical bichromatic measurement at a main and secondary wavelength,
**wherein**
a secondary wavelength above 475 nm is used at which absorption bands of haemoglobin are located.

2. Method as claimed in claim 1,
**wherein**
a secondary wavelength in the range of 546 ± 10 nm is used.

3. Method as claimed in claim 1,
**wherein**
a secondary wavelength in the range of 570 ± 10 nm is used.

4. Method as claimed in one of the claims 1-3,
**wherein**
a test is carried out which is based on a measurement of the increase or decrease of the concentration of NADH or NADPH in the sample.

5. Method as claimed in claim 4,
**wherein**
a main wavelength in the range of 340 ± 10 nm is used.

6. Method as claimed in one of the claims 1-5,
**wherein**
the content of an analyte is determined selected from the group comprising ammonia, creatine kinase and isoenzymes thereof and lactate dehydrogenase and isoenzymes thereof.

7. Method as claimed in claim 6,
**wherein**
ammonia is determined.

8. Method as claimed in claim 6,
**wherein**
creatine kinase or/and the creatine kinase isoenzyme CK-MB is determined.

9. Method as claimed in claim 6,
**wherein**
lactate dehydrogenase or/and the lactate dehydrogenase isoenzyme HBDH is determined.

10. Method as claimed in one of the claims 1-9,
**wherein**
a sample is determined which contains a blood substitute.

11. Method as claimed in one of the claims 1-10,
**wherein**
the determination is carried out on a serum or plasma sample.

12. Method as claimed in one of the claims 1-11,
**wherein**
the determination is carried out in an automated analyzer.

## Revendications

1. Procédé pour déterminer un analyte dans un échantillon contenant de l'hémoglobine libre par mesure bichromatique optique à une longueur d'onde de mesure principale et à une longueur d'onde de mesure secondaire, caractérisé en ce que l'on utilise une longueur d'onde de mesure secondaire supérieure à 475 nm à laquelle se trouvent des bandes d'absorption de l'hémoglobine.

2. Procédé selon la revendication 1 caractérisé en ce que l'on utilise une longueur d'onde de mesure secondaire dans le domaine de 546 ± 10 nm.

3. Procédé selon la revendication 1 caractérisé en ce que l'on utilise une longueur d'onde de mesure secondaire dans le domaine de 570 ± 10 nm.

4. Procédé selon l'une des revendications 1-3 caractérisé en ce que l'on réalise un test qui est basé sur une mesure de l'augmentation ou de la diminution de la concentration de NADH ou de NADPH dans l'échantillon.

5. Procédé selon la revendication 4 caractérisé en ce que l'on utilise une longueur d'onde de mesure principale dans le domaine de 340 ± 10 nm.

6. Procédé selon l'une des revendications 1-5 caractérisé en ce que l'on détermine la teneur d'un analyte choisi dans le groupe consistant en l'ammoniac, la créatine kinase et ses isoenzymes et la lactate déshydrogénase et ses isoenzymes.

7. Procédé selon la revendication 6 caractérisé en ce que l'on réalise une détermination de l'ammoniac.

8. Procédé selon la revendication 6 caractérisé en ce que l'on réalise une détermination de la créatine kinase et/ou de l'isoenzyme CK-MB de la créatine kinase.

9. Procédé selon la revendication 6 caractérisé en ce que l'on réalise une détermination de la lactate déshydrogénase et/ou de l'isoenzyme HBDH de la lactate déshydrogénase.

10. Procédé selon l'une des revendications 1-9 caractérisé en ce que l'on détermine un échantillon qui contient un produit de remplacement du sang.

11. Procédé selon l'une des revendications 1-10 caractérisé en ce que la détermination est réalisée sur un échantillon de sérum ou de plasma.

12. Procédé selon l'une des revendications 1-11 caractérisé en ce que l'on réalise la détermination dans un appareil automatique d'analyse.
